# EUROPEAN PATENT APPLICATION

(11) **EP 0 922 766 A1**
(43) Date of publication of application: **16.06.1999**
(21) Application number: 98913764.1
(22) Date of filing: 17.04.1998
(51) Int. Cl.: C12N 15/53, C12N 9/06, C12N 15/90

(54) **PROCESS FOR THE INACTIVATION OF GENES WHICH CODE FOR ENZYMES FOR THE CATABOLISM OF PHENYL ACETATE, PLASMIDS INVOLVED IN SUCH PROCESS AND STRAINS TRANSFORMED THEREWITH**

(30) Priority: 18.04.1997 ES 9700833
(71) Applicant: Antibioticos, S.A., 28036 Madrid (ES)
(72) Inventor: FERNANDEZ CANON, José Manuel, E-28002 Madrid (ES); RODRIGUEZ SAIZ, Marta, E-36209 Vigo (ES); DIEZ GARCIA, Bruno, E-24192 Leon (ES); BARREDO FUENTE, José Luis, E-24006 Leon (ES); VITALLER ALBA, Alejandro, E-24001 León (ES); SALTO MALDONADO, Francisco, E-28023 Madrid (ES); PENALVA SOTO, Miguel Angel, E-28036 Madrid (ES); MINGOT ASCENCAO, Jose Manuel, E-28036 Madrid (ES)
(74) Representative: Elzaburu Marquez, Alberto
(86) International application number: ES9800101
(87) International publication number: WO9848019

(57) **Abstract**

Procedure for the inactivation of genes that code for phenylacetate catabolism enzymes, plasmids containing them, and strains transformed with them. The procedure is preferably applied to the *phacA* gene of *A. nidulans* and to the *pahA* gene of *P. chrysogenum*, genes which code respectively for enzymes competing for this substrate with penicillin biosynthesis enzymes. The non-expression of these enzymes has a beneficial effect on the synthesis of this antibiotic, increasing its yield, with a lower phenylacetate demand per unit of culture.

## Description

### Field of the invention

The present invention is based on the characterization of new DNA compounds and recombinant DNA molecules that code for the enzyme phenylacetate 2-hydroxylase obtained from the filamentous fungi *Aspergillus nidulans* and *Penicillium chrysogenum*. The transformation of penicillin-producing strains with the aforesaid DNA compounds allows production of this antibiotic to be increased.

### Prior art

The last step of the benzylpenicillin (penicillin G) biosynthesis pathway in certain filamentous fungi consists in the conversion of isopenicillin N into penicillin G. This involves a transacylation reaction, in which the side chain of L-aminoadipate in isopenicillin N is replaced by one of phenylacetic acid. The enzyme that catalyses this reaction (acyl-CoA:isopenicillin N acyltransferase) uses activated phenylacetic acid in the form of a thioester of coenzyme A (CoA) as one of its substrates.

*Penicillium chrysogenum*, the fungus used for the industrial production of penicillin, and *Aspergillus nidulans*, are unable to synthesize phenylacetate. Consequently, to facilitate the biosynthesis of penicillin G, it is necessary to add a phenylacecate excess to the industrial *P. chrysogenum* cultures. This excess, which prevents the unwanted biosynthesis of other penicillins with aliphatic side chains, increases the final costs of the fermentation process

Part of the phenylacetate added to the penicillin production cultures can be metabolized. It is a fully established fact that a considerable quantity of 2-hydroxyphenylacetate is accumulated in the *P. chrysogenum* fermentations. This side chain precursor fraction obviously does not contribute to the biosynthesis of penicillin G.

Both *Aspergillus nidulans* and *P. chrysogenum* synthesize penicillin from three precursor amino acids via the same enzymatic steps. Moreover, like *P*. *chrysogenum*, *A*. *nidulans* converts a phenylacetate fraction into 2-hydroxyphenylacetate when the first compound in excess is added to the cultures. Besides converting the phenylacetate into 2-hydroxy-phenylacetate, *A. nidulans* can also catabolize this last compound and, in fact, it is able to use phenylacetate as the sole carbon source.

The catabolic pathway in *A. nidulans* is described in the following diagram:
Phenylacetate
   ↓ phenylacetate hydroxylase
2-hydroxyphenylacetate
   ↓ 2-hydroxyphenylacetate hydroxylase
Homogenizate
   ↓ homogenizate dioxygenase
Maleylacetoacetate
   ↓ maleylacetoacetate isomerase
Fumarylacetoacetate
   ↓ fumarylacetoacetate hydrolase
Fumarate + Acetoacetate

The ortho-hydroxylation of phenylacetate is the first step in this pathway. A second hydroxylating reaction converts 2-hydroxyphenylacetate into 2,5-dihydroxyphenylacetate (= homogenizate). The homogenizate is catabolized via fumarylacetoacetate into fumarate and acetoacetate, which are incorporated into the Krebs cycle (see Fernández Cañón y Peñalva, Proc. Natl. Acad. Sci. USA 92:9132-9136, 1995).

The ability of the said filamentous fungi to catabolize phenylacetate partially or completely is a detrimental characteristic in the production of penicillin. For this reason, the total or partial elimination of this characteristic would result in strains with improved penicillin G production capacity.

The elimination of this detrimental characteristic (namely the ability to catabolize phenylacetate partially or completely) by classical mutagenesis in penicillin-producing fungi would require enormous selection efforts in a large number of strains. In any case, the said mutagenesis frequently gives rise to second mutations which might eliminate useful characteristics of the parental strains, producing, for example, auxotrophic mutations or mutations which decrease the vigour of growth or spore formation that are critical for industrial fermentation. It is appropriate therefore to use genetic engineering techniques, which do not have the said limitations, to eliminate the ability to catabolize phenylacetate partially or completely. An essential requirement for carrying out directed genetic engineering is the cloning and characterizacion of the gene(s) which mediate the aforesaid detrimental characteristic of the penicillin-producing fungi.

### Detailed description of the invention

It is the object of the present invention to solve the aforesaid existing problems of the State of the Art. It consists in characterizing a fungal gene which codes for a phenylacetate 2-hydroxylase activity, the enzyme that catalyses the first enzymatic step of phenylacetate catabolism in *Aspergillus* and *Penicillium*, and its use to eliminate the gene present in the genome of the penicillin-producing fungi by means of recombinant DNA technology. The present patent describes how this gene is inactivated in a strain constructed by genetic engineering. This strain, which is unable to catabolize phenylacetate, produces higher levels of penicillin than the parental strain. In addition, the maximum penicillin production of this recombinant strain requires a smaller phenylacetate excess than is needed by the parental strain. The inactivation of phenylacetate catabolism using the above DNA compound(s) results in a significant improvement in the production of penicillin.

The sequence of a genomic DNA fragment of 1986 base pairs (bp) from *A*. *nidulans* that includes the new gene used for the present invention is shown in SEQ ID NO:1. The gene was called *phacA* (phac because of the use of phenylacetate) and codes for an enzyme that orthohydroxylates phenylacetate (this reaction being the first step of phenylacetate catabolism in *A. nidulans*). The nucleotide sequence of the complementary DNA (cDNA) clones that cover the complete coding region and their subsequent alignment with the genomic DNA sequence revealed the following characteristics of this gene:
- The gene codes for a polipeptide of 518 amino acids. The first methionine is encoded by an ATG triplet at position 82, while the end codon (TAG) is located at position 181C. These positions match the nucleotide sequence that is shown in SEQ ID NO: 1.
- The coding region is interrupted by three introns 65, 56 and 53 nucleotides in length (SEQ ID NO: 1).
- The deduced polypeptide of 518 residues is shown in the three-letter amino acids code below its corresponding exons in SEQ ID NO:1 and also, in an isolated context, in SEQ ID NO: 2. The molecular weight of the deduced protein is 58,495 g/mol. A search carried but using the public access server BLAST of the National Center for Biotechnology Information (NCBI, USA) in the databases of protein sequences (e.g. SwissProt and PIR) and in the conceptual translation of databases of DNA sequences (e.g. the GenBank and EMBL databases) in the six possible reading frames showed that the amino acid sequence was similar to members of the cytochrome P450 family (haemothiolated protein). These proteins are usually involved in oxidative processes. In fact, the sequences between residues 431 and 439 correspond to the peptide which contains the amino acid cysteine, Gly-X-Gly-X-X-X-Cys-X-Gly (where X indicates any amino acid), which is involved in the binding of the haem group that is characteristic of these haemothiolated proteins.

The new DNA compound, the structure of which is described in detail in SEQ ID NO:1 and which was isolated from a natural microorganism, can be completely synthesized using automatic DNA synthesizers. In addition, owing to the degenerate nature of the genetic code, the protein encoded by the new DNA compound could be encoded by alternative DNA sequences. These are included in the present invention. On the other hand, any natural genetic variant derived from the new DNA compound described above is considered equivalent thereto. These genetic variants include homologous genes in organisms closely related to *A*. *nidulans* in evolutionary terms, such as *P. chrysogenum*. These genes can be easily identified using the DNA compound from *A. nidulans* as a hybridization probe, as described below.

The *phacA* gene of *A. nidulans* can be used as a molecular probe to search for functional homologous genes in genomic DNA or cDNA libraries for other fungal species by hybridization. Thus, for example, the present patent describes its use for screening a *P*. c*hrysogenum* DNA library, showing that the gene homologous to the *phacA* of *A. nidulans* can be isolated from industrial strains of *P. chrysogenum*. The sequence of a genomic DNA fragment of 2558 bp from *P*. *chrysogenum* isolated by hybridization with a *phacA* gene probe is shown in SEQ ID NO: 3. The *P. chrysogenum* gene homologous to the *phacA* of *A. nidulans* was designated *pahA* (for phenylacetate hydroxylation). The *pahA* gene of *P. chrysogenum* codes for a polypeptide of 516 amino acids which shows 84% identity with the amino acid sequence of *PhacA*, the protein product of the *phacA* gene of *A. nidulans*. The sequence of the product of the *pahA* gene of P*. chrysogenum* is shown in SEQ ID NO:4. As has been indicated earlier, this new DNA compound isolated from *P. chrysogenum* is also included in the present invention, as are the other homologous genes which can be isolated from other species of fungi by similar procedures.

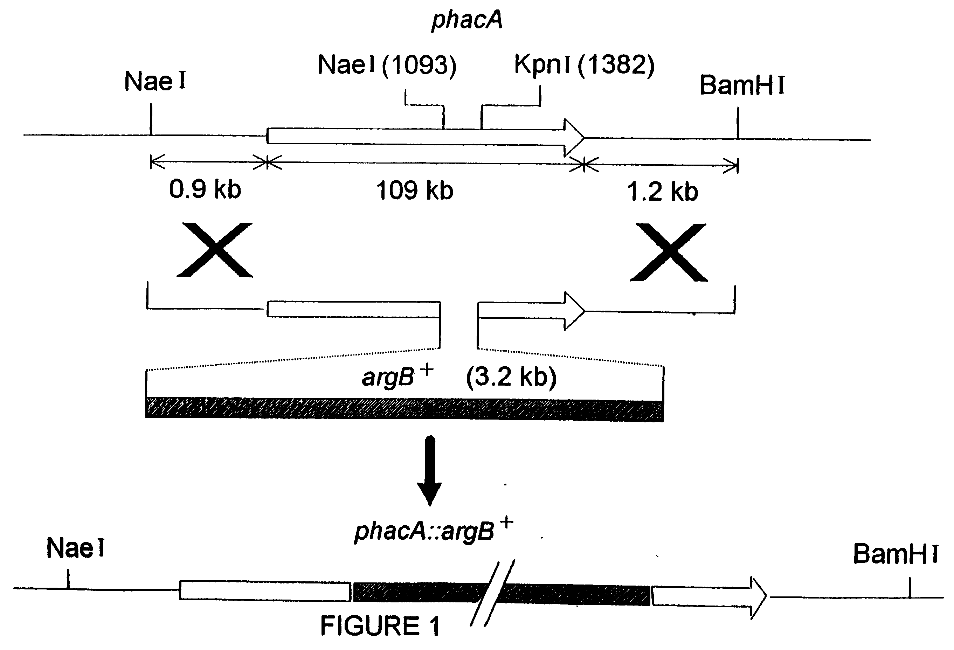

The cloned genes can be used to generate loss-of-function mutations by reverse genetics. For this purpose a new recombinant DNA molecule can be constructed in an *Escherchia coli* vector carrying a truncated version of the fungal gene, which can be used to inactivate the endogenous gene by transformation. For example, this recombinant plasmid can contain the 5' region of the p*hacA* gene of *A. nidulans*, followed by a modification in the coding region of the *phacA* gene which consists in the replacement of the 289 bp *NaeI*-*KpnI* fragment by a 3.2 kb *XbaI* fragment which contains the *argB*^{*+*} gene of *A. nidulans* (see Fig. 1). The 3' region of the *phacA* gene is then made available. The expression of this mutant *phacA* gene would result in a PhacA protein that is truncated at residue 297 and consequently lacks 221 carboxyterminal residues. The latter residues, absent in the mutant protein, include the abovementioned peptide which includes the Cys residue that is involved in the binding of haem groups and is essential for the activity of proteins of this type.

A linear DNA fragment containing the above-mentioned regions can be separated from the sequences of the vector by means of suitable restriction enzymes and then purified by standard techniques. This linear fragment can be used for the transformation of an *argB*^{*+*} strain of *A. nidulans* into an arginine prototroph. As the DNA used in the transformation lacks the sequences required for its autonomous replication, the prototrophic transformants result from the integration of the DNA fragment in the genome. One of the possible methods of integration which would give rise to an *argB*^{*+*} phenotype would be via double intercrossing between the linear DNA molecule and the resident phacA locus in the genome of the fungus, as shown in Fig. 1. This double intercrossing event results in the replacement of the original phacA gene by the truncated gene generated in vitro, causing the loss of the *phacA* function. The transformants carrying this substitution (i.e. with the loss-of-function mutation) can be recognized by their hybridization pattern when their genomic DNA is digested with the appropriate restriction enzymes and analysed by hybridization with the Southern technique, using as probes the *phacA* or *argB* genes radioactively labelled with ³²P. In this way, the hybridization patterns of other types of transformants can be easily distinguished from those corresponding to a gene substitution. The transformants which lack the function provided by the *phacA* gene can be purified for further assays using standard techniques.

Unlike the wild-type strain, the *A. nidulans* transformants produced by reverse genetics which lack the function of the *phacA* gene cannot grow in phenylacetate as the sole carbon source. They do grow in 2-hydroxyphenylacetate or 2,5-dihydroxyphenylacetate, however. This shows that the new DNA compound (the *phacA* gene of *A. nidulans*) codes for an enzyme activity needed for the orthohydroxylation of phenylacetate, an essential step for the utilization of phenylacetate in *A. nidulans*. *P. chrysogenum* does not use phenylacetate as the sole carbon source but, as shown earlier, contains a gene which codes for a homologue of the enzyme *PhacA*. The new DNA compound, therefore, provides a method for eliminating lateral metabolic conversions which can decrease the accumulation of phenylacetate available for penicillin biosynthesis.

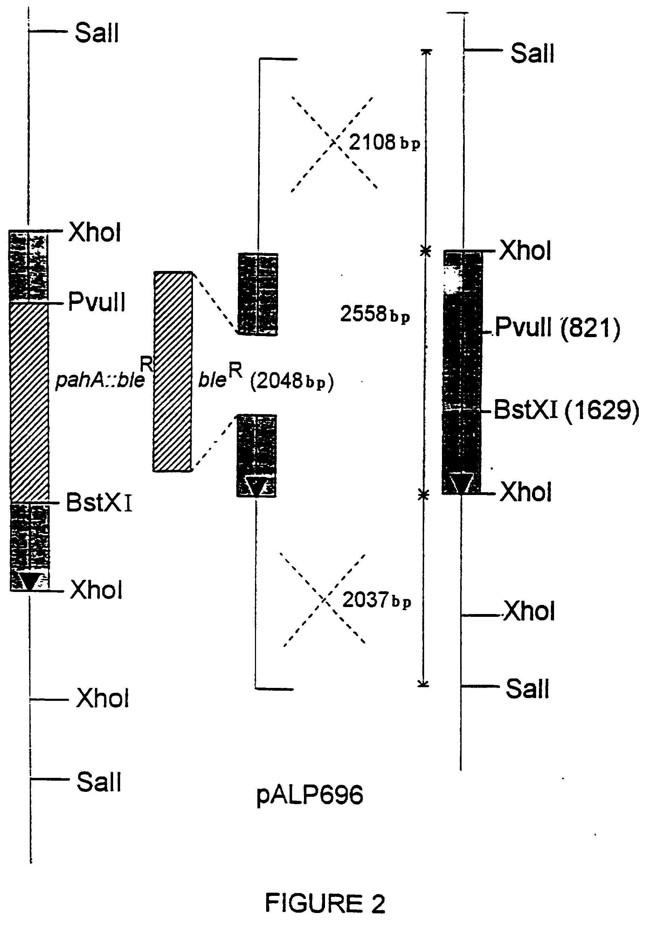

In *P. chrysogenum* a methodology similar to that described can be used to inactivate the *pahA* gene, using the new *P. chrysogenum* DNA compound included in this invention and any of the transformation markers already available for *P. chrysogenum,* for example the *trpC* gene. It will also be appreciated that transformation markers other than a*rgB* (in *A. nidulans*) or *trpC* (in *P. chrysogenum*) could be used to interrupt the coding region of the new DNA compound. These include other auxotrophic markers (*pyrG* or *riboB* for example) and antibiotics-resistant genes (for example the genes which confer fleomycin or hygromycin B resistance in fungi). Such methods, basically equivalent to those used here, are also included in the present invention. Fig. 2 shows one of these methods, which can be used to change the *pahA* gene of *P*. *chrysogenum*. In this case, in the inactivation construct, an internal region of the *pahA* gene has been replaced by a chimeric gene where the promoter of the *gdh* gene of *P. chrysogenum* (a gene which codes for glutamate dehydrogenase enzyme activity) controls the expression of the bacterial *ble*^{*R*} gene, which confers resistance to the antibiotic fleomycin. The plasmid which includes the inactivation construct is called pALP696. The transformants can therefore be selected for their ability to grow in media containing fleomycin (Kolar, M., Punt, P.J., van del Hondel, C.A.M.J.J. and Schwab, H. Gene 62: 127-134, 1988).

In addition, other strategies can be used to generate loss-of-function mutations by reverse genetics in a gene which has been cloned and characterized. For example, transformation with a circular plasmid carrying an internal fragment of the target gene, after homologous integration of a single copy of the transformant DNA, gives rise to two incomplete copies of the gene, which lack functionality, if the fragment included in the plasmid is suitably chosen. The methods that use these or other strategies to generate a loss-of-function mutation in the desired gene by reverse genetics are also included in this invention.

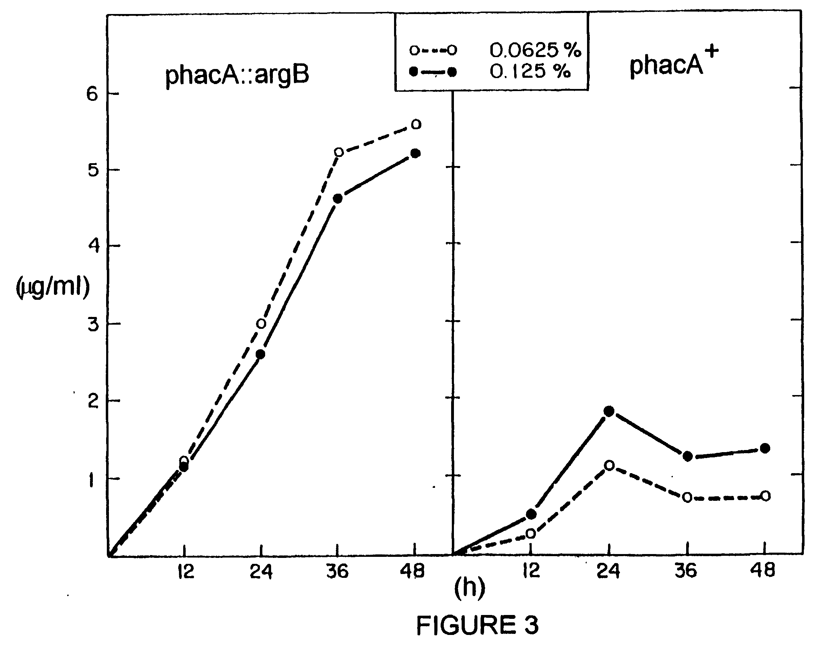

The fungal strains in which the enzymes involved in the modification or catabolism of phenylacetate have been eliminated show improved penicillin production properties. Nevertheless, their ability to grow in penicillin production media is indistinguishable from that of the parental strain. For example, the *A. nidulans* transformants with the *phacA* gene substitution described above (i.e. lacking phenylacetate 2-hydroxylase activity) produce 3 to 8 times more penicillin than the parental strain and are less dependent on the amount of phenylacetate added (see Fig. 3). In this way, whereas the reduction of phenylacetate from 0.125% (w/v) to 0.0625% (w/v) results in a significant drop in penicillin yields in the wild strain, the transformants which lack the function of the *phacA* gene produce high levels of penicillin with both phenylacetate concentrations.

Finally, this invention does not only concern the *phacA* gene and its homologues. We are here using the gene that codes for phenylacetate 2-hydroxylase because this enzyme catalyses the first step in the phenylacetate catabolic pathway and the *P. chrysogenum* production strains secrete large quantities of 2-hydroxyphenylacetate. Once they have been characterized, other genes involved in phenylacetate metabolism could be inactivated using a method similar to the one described here. This invention, i.e. that penicillin yields from fungal organisms can be improved by inactivating individual phenylacetate metabolism genes using reverse genetics, also covers these alternative possibilities.

### Examples

### EXAMPLE 1. CLONING AND CHARACTERIZATION OF THE phacA GENE OF A. nidulans.

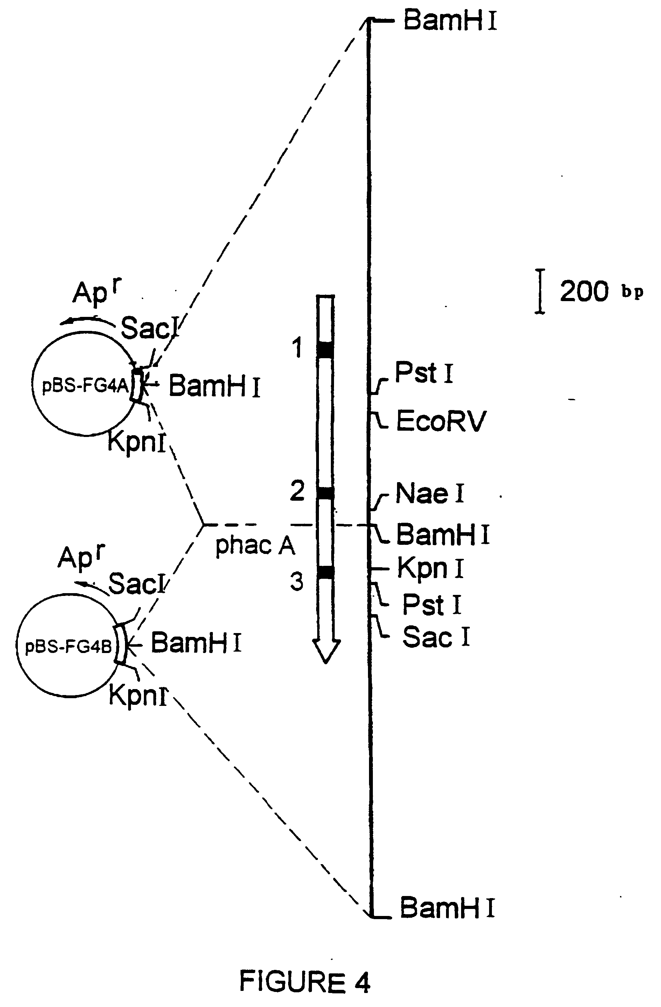

The cDNA clones corresponding to the *phacA* gene were isolated by differential selection from a cDNA library, taking the steps described below:
a) Obtaining a cDNA population corresponding to transcripts of genes which are preferentially expressed when the fungus is grown in the presence of phenylacetate (for use as a "plus" probe in screening the DNA library).
   The *A. nidulans* A26 strain from the Fungal Genetics Stock Center collection (Department of Microbiology, Medical Center of the University of Kansas) was cultured at 37°C in a suitably supplemented minimal medium containing (in g/l) KPO₄H₂, 13.6; (NH₄)₂SO₄, 2.0; MgSO₄ x 7H₂O, 0.25 and Fe x 7H₂O, 0.0005, with 0.3% (w/v) glucose as carbon source. The time when the glucose was used up was determined by analysing the glucose concentration in the culture medium (using an enzyme kit). This was usually after 18 hours of incubation with constant agitation. Two hours later phenylacetate was added to the culture, to a final concentration of 10 mM, and the culture was agitated at 37°C for 1 hour longer with the aim of inducing the expression of the stated genes. After this amount of time, the mycelium was collected by filtration, washed with water, frozen in liquid nitrogen, lyophilized and used to isolate total RNA. This total RNA preparation was used to isolate poly(A⁺) mRNA by oligo-dT cellulose affinity chromatography. Single-stranded cDNA was prepared in the presence of avian myeloblastosis virus reverse transcriptase (of commercial origin), using as a template 2 µg of the said mRNA isolated from mycelium induced with phenylacetate and oligo-dT (15-mer) as primer. The reaction was incubated for 1 hour at 42°C in a buffer containing 10 mM Tris-HCl, pH 8.8 (at 25°C), 50 mM KCl, 0.1% Triton X-100, 5 mM MgCl₂, 10 mM of each dNTP and 0.5 units of RNasin. After first strand synthesis, the template RNA was eliminated by incubation for 1 hour at 60°C with 3M NaOH. After neutralization (with acetic acid), the single-stranded DNA was recovered by centrifugation by means of precipitation with 2.5 volumes of absolute ethanol for 2 hours at -80°C. This cDNA was removed with a 30-fold excess of poly(A⁺) RNA isolated from the mycelium recovered at the time when the glucose was used up, using the procedure described by Sargent, T.D., Methods Enzymol. 152: 423-432, 1987. The cDNA-RNA hybrid molecules were separated by hydroxyapatite chromatography at 68°C and discarded. The remaining cDNA was hybridized as stated, with an excess of poly(A⁺) RNA from mycelium cultured in the absence of phenylacetate. The cDNA-RNA hybrid molecules were discarded again, as stated, and the resultant single-stranded cDNA population, which represented transcripts of genes preferentially expressed in the presence of phenylacetate, was collected. This cDNA was uniformly labelled with [α⁻³²P]dCTP and excess of all the remaining dNTPs, in the presence of the Klenow fragment of the DNA polymerase and random-sequence hexanucleotides as primers. The population of ³²P-labelled cDNAs obtained (specific activity > 10⁸ cpm/mg) was used as a probe to screen a cDNA library constructed as described in Section c).
b) Obtaining a cDNA probe corresponding to genes transcribed in the absence of phenylacetate ("minus" probe).
   Single-stranded cDNA was synthesized and labelled with ³²P as described in Section a), but using mRNA from mycelium which was cultured until the glucose of the culture medium was used up and incubated for 1 hour longer at 37°C in the absence of phenylacetate.
c) Construction of a cDNA library in the λgt10 vector, using cDNA obtained from mycelium cultured in the presence of 10 mM phenylacetate as sole carbon source.
   To construct this cDNA library, the first strand cDNA synthesis reaction was carried out as described earlier. The cDNA-RNA hybrid obtained was converted into double-stranded cDNA by introducing random breaks in the RNA strand with RNase H, which were used as primer points by the *Escherichia coli* DNA polymerase I. To add E*coRI* ends to this blunt-ended cDNA preparation, synthetic adaptors which contained the blunt phosphorylated end and the *EcoRI* overhang with the double-stranded cDNA were incubated in the presence of T4 DNA ligase. The cDNAs of the *EcoRI* end were then purified and phosphorylated with T4 polynucleotide kinase and ATP. The phosphorylated cDNA was mixed with the arms of the λgt10 vector, which had been previously digested with *EcoRI* and dephosphorylated, and the mixture was incubated with T4 DNA ligase. The recombinant DNA molecules were packaged in vitro, using commercial packaging extracts. The recombinant phages in which a cDNA insert had inactivated the *cI* gene (in which the *EcoRI* site is present) were selected for their lytic phenotype in an *E. coli hfl* strain (F-, *thi-1*, *thr-1*, *leuB6*, *lacY1*, *tonA21*, *supE44*, *hflA150*, [chr::Tn10]. In this way a total of 10⁷ recombinant clones were obtained.
d) Screening of the cDNA library.
   The cDNA library was plaqued out using the *E. coli* C600 hfl⁺ strain and the lysis plaques obtained were transferred in duplicate to nitrocellulose filters. One of the replicas was hybridized with the "plus" cDNA probe (described in a)) and the second replica was hybridized with the "minus" cDNA probe (described in b)). The clones which hybridized with the "plus" probe and did not hybridize with the "minus" probe were then selected and purified.
e) Identification of the cDNA corresponding to the *phacA* gene.
   The cDNA inserts were excised from the vector by digestion with the endonuclease *NotI*, using the *NotI* cleavage sites included in the *EcoRI* adaptor. The inserts of the selected clones were subcloned in pBluescript SK(+) plasmid digested with *NotI* and then they were sequenced by standard procedures. The sequences of the cDNA inserts were translated in the six possible reading frames and compared using the BLAST algorithm with the conceptual translation in the six reading frames of the public access DNA sequence databases GenBank and EMBL or with the protein sequence databases SwissProt and PIR. One of the sequences of the cDNA inserts generated an extensive open reading frame, not interrupted by termination codons. The deduced amino acid sequence showed significant identity with the amino acid sequences of proteins in the cytochrome P450 family, which are usually involved in oxidation reactions. As will be shown later (see Example 3), the open reading frame codes for a phenylacetate 2-hydroxylase. This cDNA insert was used to obtain new cDNA clones corresponding to this gene by hybridization. These clones were sequenced through both strands and the nucleotide sequence obtained showed a complete open reading frame 1554 bp in length (without the termination codon of the translation) which codes for a polypeptide of 518 amino acids (SEQ ID NO: 2). The identity of this sequence with members of the cytochrome P450 protein family unequivocally established that this deduced protein represents a new member of this family.
f) Cloning of the *phacA* gene.
   An *A*. *nidulans* genomic DNA library constructed in the λEMBL4 vector was screened with a ³²P-labelled cDNA probe corresponding to the almost-complete transcript. The positive clones were purified and their inserts were characterized by digestion with restriction enzymes and hybridization with the above-mentioned probe. In this way the hybridization zone of two contiguous *BamHI* fragments 2.4 and 1.9 kb in length was mapped. Fig. 4 shows a restriction map of the genomic region which contains these fragments. The said *BamHI* fragments were subcloned in pBluescript SK(+). The recombinant plasmid which contained the 2.4 kb *BamHI* fragment was designated pBS-FG4A, while the plasmid which carried the 1.9 kb *BamHI* fragment was designated pBS-FG4B.

### EXAMPLE 2. CLONING AND CHARACTERIZATION OF THE pahA GENE OF P. chrysogenum, WHICH IS A FUNCTIONAL HOMOLOGUE OF THE phacA GENE OF A. nidulans.

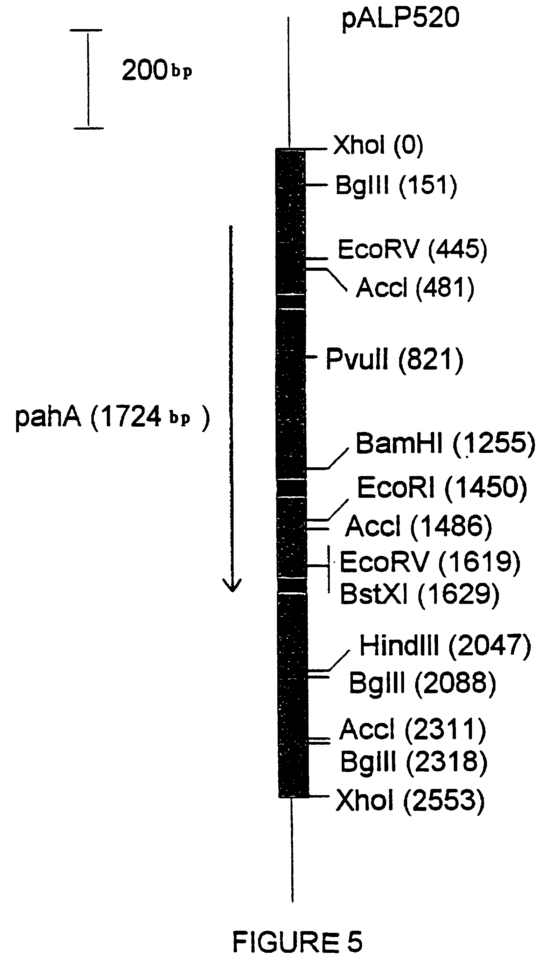

a) Cloning and determination of the nucleotide sequence of the *pahA* gene of P. c*hrysogenum*.
   A *P. chrysogenu*m Wisconsin 54-1255 genomic DNA library, constructed with partial Sau3A fragments cloned in the *BamHI* site of λEMBL4, was screened with a (³²P) radioactively labelled probe consisting of a cDNA of the almost complete length of the *phacA* gene. Around 12,000 clones were transferred to cellulose filters by means of standard techniques. They were then hybridized for 24 h at 37°C in a buffer which contained 50% of formamide, 5 x Denhart's solution, 5 x SSC, 0.1% of SDS, 50 µg/ml of sonicated herring sperm single-stranded DNA and 50 ng of the labelled probe. The final washing of the hybridized filtrates was carried out in 0.2 x SSC, 0.1% SDS at 37°C. Ten clones were purified by this procedure and their DNA was isolated. The inserts of the said clones were then characterized by digestion with restriction enzymes and hybridization with the aforesaid probe. The hybridization region was located in a 2.55 kb *XhoI* fragment, the restriction map of which is shown in Fig. 5. This DNA fragment was subcloned in the plasmid pBluescript SK(+), giving rise to the plasmid called pALP520. The nucleotide sequence of th*e XhoI* fragment which included the *pahA* gene (Fig. 5) was determined by the dideoxynucleotide method (Sanger, F., Nicklen, S. and Coulson, A.R. (1977) Proc. Natl. Acad. Sci. U.S.A. 74, 5463-5467).
b) Construction of a *P. chrysogenum* cDNA library from purified RNA under penicillin production conditions. Characterization of the cDNA corresponding to the *pahA* gene.
   The mycelium from fermenter cultures of an industrial strain of *P. chrysogenum*, grown under penicillin G production conditions, was collected after 100, 124 and 148 hours of incubation and used to obtain poly(A⁺) mRNA with the Poly (A) Quick mRNA Purification Kit (Stratagene). This poly(A⁺) mRNA was used as a template for the construction of a cDNA library using the Zap-cDNA Synthesis Kit (Stratagene), in accordance with the manufacturer's instructions. The double-stranded cDNA was inserted between the *EcoRI* and *XhoI* restriction sites of the phage vector λZAP, the 5' region of the transcripts being situated at the side of the *EcoRI* site. The cDNA library was packaged with Gigapack II Gold packaging extracts (Stratagene), generating about 10⁶ independent clones.

The screening of this cDNA library was carried out by standard procedures (Sambrook, J. Fritsch, E.F. and Maniatis, T. (1989), Molecular Cloning: A Laboratory Manual, 2^{nd} ed., Cold Spring Harbor Laboratory Press, New York), using a 1174 bp *EcoRV* fragment within the *pahA* gene as a probe (Fig. 5). In this way twelve positive recombinant clones (called #1 to #12) were isolated, and the clone with the insert of greatest size (approximately 1600 bp) was chosen for characterization. The complete nucleotide sequence of this clone was then determined, it being observed that it included an open reading frame (ORF) of 1548 bp, which codes for a polypeptide of 516 amino acids with a molecular weight of 58,112 Da and 84% identity with the amino acid sequence of the protein encoded by the *phacA* gene of *A. nidulans*. As was the case with its homologous *A. nidulans* gene, investigation of the databases showed considerable identity between the *PahA* protein and members of the P450 protein family. The *PahA* protein contains the sequence Gly-X-Gly-X-X-X-Cys-X-Gly (residues 430-438, SEQ ID NO: 4) which characterizes this type of protein (see the Detailed Description of this invention). From these results it is concluded that the *pahA* gene of *P. chrysogenum* is the homologue of the *phacA* gene of *A. nidulans.*

### EXAMPLE 3. INACTIVATION OF THE phacA GENE OF A. nidulans BY REVERSE GENETICS.

The wild allele of the *phacA* gene of *A. nidulans* was replaced by a mutant allele in which part of its coding region, presumably essential for its function residues 298 to 392 in SEQ ID NO: 1), had been suppressed, being replaced by the *argB*^{*+*} gene. This genetic manipulation thus truncates the *phacA* gene at the codon 297, producing a null mutant allele.

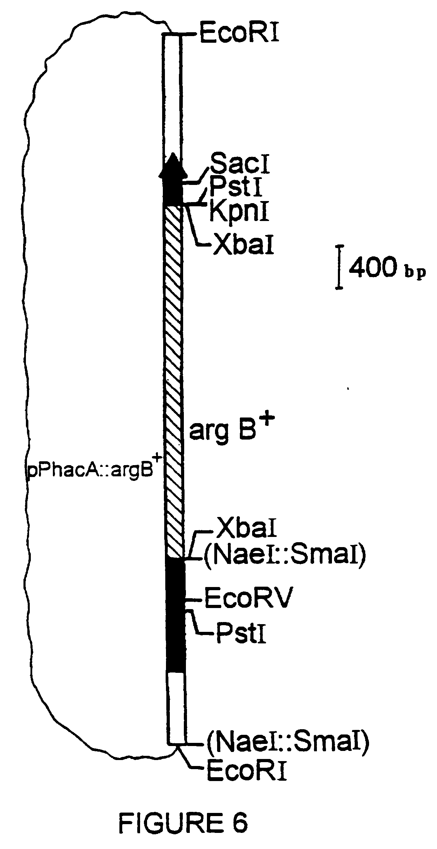

Moreover, a 1.7 kb *KpnI*-*EcoRI* fragment was purified from pBS-FG4B (Fig. 4) and subcloned in pUC18 digested with these two enzymes, giving rise to the plasmid pUCB. A 1.9 kb *NaeI* fragment was then purified from pBS-FG4A (Fig. 4) and cloned in pBluescript SK (+; digested with *SmaI*. A plasmid was selected in which the coding strand of the incomplete *phacA* gene (starting at the *NaeI* site) was found in the same orientation as the gene coding for β-galactosidase in the vector. This plasmid was called pBSA. An *XbaI-HindIII* DNA fragment was purified from pBSA and inserted into pUCB digested with *XbaI-HindIII*, giving rise to the plasmid pUCA-B. Finally, a 3.2 kb DNA fragment containing the *argB*⁺ gene of *A. nidulans* was inserted at the only *XbaI* site of pUCA-B, to give pPhacA::argB⁺. Starting from the *lacZ* promoter of pUC18, pPhacA::argB includes an 0.94 kb fragment of the 5' region of the *phacA* gene, followed sequentially by the first 297 codons of its genomic sequence, the 3.2 kb fragment containing the *argB* gene, the genomic sequence of the *phacA* gene corresponding to the codons 393-518 and 1.2 kb of the 3' region of the *phacA* gene. A linear fragment containing all these regions can be purified from the plasmid by *EcoRI* digestion (Fig. 6).

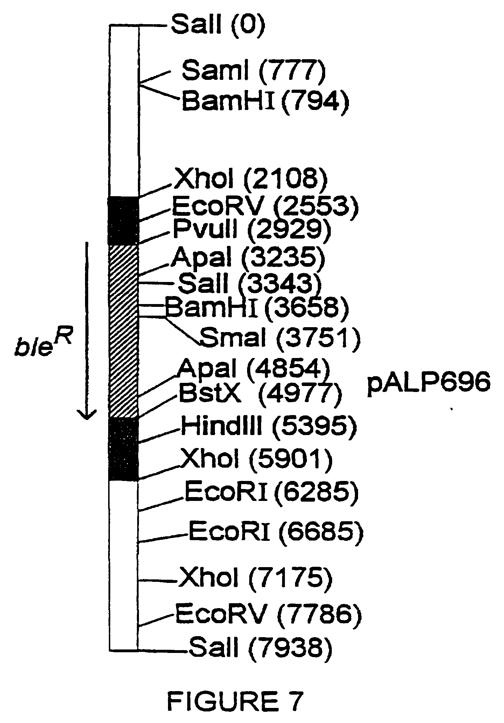

Likewise, transformation can be used to construct a strain of *A. nidulans* which includes a disruption-deletion mutation in the *phacA* gene using this linear *EcoRI* fragment and employing the strategy summarized in the diagram described above. For this purpose, protoplasts of the strain of *A. nidulans* biA1, *methG1*, *argB2* were transformed with 2 µg of the said DNA fragment, using the protocol of Tilburn, J., Scazzocenio, C., Taylor, G.G., Zabicky-Zissman, J.H., Lockington, R.A. and Davies, R.W. (1983) Gene 26: 205-211. The transformants were selected for arginine prototropny using a suitable selective medium and were then purified. DNA was isolated from the mycelium corresponding to a series of transformants, digested with *PstI* and analysed by Southern hybridization, using specific *phacA* and *argB* gene probes. A number of transformants showed the hybridization pattern which it was hoped to obtain for double intercrossing to be successful, as shown in Fig. 1. For example, using the 0.9 kb *PstI* fragment of the *phacA* gene as a probe (see Fig. 4), it was found that the individual 0.9 kb *PstI* hybridization band of the receptor strain had been replaced by a 3.8 kb band in the transformants which exhibited the type of integration shown in Fig. 1. This band also hybridized with the *argB* probe. Two of these transformants were selected for subsequent characterization and were designated Δ*phacA* #1 and Δ*phacA* #2. These transformed strains had the ability to grow in culture media with phenylalanine, 2-hydroxyphenylacetate or 2,5-dihydroxyphenylacetate as sole carbon source but on the contrary, they did not grow in culture media with phenylacetate as sole carbon source. This supports the conclusion that the enzyme cytochrome P450 encoded by the *phacA* gene of *A. nidulans* (and by extension the one encoded by the *pahA* gene of *P. chrysogenum*) has an activity which hydroxylates phenylacetate to 2-hydroxyphenylacetate, this activity catalysing the first step in the phenylacetate utilization pathway in *A. nidulans* (see Prior Art). The *A. nidulans* strain Δ*phacA* #l, which was phenotypically indistinguishable from the strain Δ*phacA* #2, was deposited in the Spanish Collection of Type Cultures (CECT), University of Valencia, Research Building, Burjasot Campus, 46100, Valencia, on 19 June 1996, as *A. nidulans biAl veA1 methG1 argB2 phacA* :: [pPhacA :: argB⁺] with the access number CECT 20195. The obtaining of *P. chrysogenum* transformants with the inactivated *pahA* gene is very obvious for any person skilled in the art. It would simply involve isolating the *pahA* gene of *P. chrysogenum* by hybridization with the *phacA* gene of *A. nidulans* present in the transformant CECT 20195 or alternatively by hydbridization with synthetic oligonucleotides based on SEQ ID No:1. Subsequently, using the plasmid pALfleo7 (deposited in the Spanish Collection of Type Cultures (CECT), University of Valencia, Research Building, Burjasot Campus, 46100, Valencia, on 20 February 1997, as CECT 4849), the plasmid pALP696 would be constructed as shown in Fig. 7 of the present patent.

### EXAMPLE 4. EXPERIMENT TO INACTIVATE THE FUNCTION OF THE phacA GENE AND INCREASE PENICILLIN YIELD.

To find out whether an improved yield of penicillin would be obtained by eliminating the phenylacetate 2-hydroxylase activity encoded by the *phacA* gene, experiments were carried out to measure the levels of penicillin produced by the strain Δ*phacA* in comparison with a strain *phacA*^{*+*}. Both strains were compared by fermentation in a flask in a minimal medium which contained 2.5% (w/v) of lactose as principal carbon source and 2.5% (w/v) of corn steep solid, with different quantities of sodium phenylacetate (in w/v) as indicated. The cultures were inoculated in all cases with an equal number of viable conidiospores (2 x 10⁶/ml) and incubated at 37°C with orbital agitation (250 r.p.m.). Samples were taken at various times and filtered through Miracloth, and the supernatants were used to measure the penicillin produced by bioassay.

For the bioassay 1 ml of a *Micrococcus luteus* culture grown up to a DO⁶⁰⁰ = 6 was mixed with 1 l of the medium Antibiotic No. 1 (Difco) at 50°C. This mixture was poured into 13.6 cm Petri dishes (65 ml/dish). 100-µl samples corresponding to the supernatants from the cultures (suitably diluted in 10 mM sodium phosphate buffer, pH 6,8) were arranged in 8 mm diameter wells. The plates were incubated for 2 hours at 4°C and then for a further 22 h at 37°C. After this, the diameter of the bacterial growth inhibition zones caused by the penicillin present in the samples was measured and the amount of antibiotic was estimated by comparing with a calibration line produced with different amounts of penicillin G potassium.

Fig. 3 shows that the highest levels of penicillin corresponding to the control strain *phacA*^{*+*} were obtained after 24 hours using 0.125% of phenylacecate in the production medium. The yield achieved at this time was 1.8 µg/ml penicillin. Halving the phenylacetate concentration reduced the maximum levels of penicillin produced by this strain to 1.1 µg/ml.

Fig. 3 also shows that, in marked contrast, the highest levels of penicillin in a culture of the strain Δ*phacA* reached 5.6 µg/ml, i.e. levels 3.1 times higher than those of its parental strain. Moreover, as a result of the inactivation of the *phacA* gene, the increase in penicillin production was not affected by the reduction of the initial phenylacetate concentration to 0.0625%.

It is concluded that a disruption-deletion mutation of the *phacA* gene which codes for a phenylacetate 2-hydroxylase activity, carried out by means of reverse genetics techniques as shown in Fig. 1, gives at least two advantages in the production of penicillin by *A. nidulans*: (i) penicillin levels are considerably increased and (ii) penicillin production is less dependent on the external supply of phenylacetate.

### Detailed description of the figures.

Fig. 1: Strategy used to produce a disruption-deletion mutation in the *phacA* gene of *A. nidulans* by homologous recombination. The putative intercrossings are shown by big crosses in bold.
Fig. 2: Strategy used to produce a disruption-deletion mutation in the *pahA* gene of *P. chrysogenum* by homologous recombination. The putative intercrossings are shown by big broken-line crosses.
Fig. 3: Penicillin yield of a transformed phacA::argB strain of *A. nidulans* in comparison with a wild-type strain (*phacA*^{*+*}). Abscissae: time in hours (h); Ordinates: penicillin yield (µg/ml). Continuous line: Phenylacetate concentration 0.125%; Broken line: Phenylacetate concentration 0.0625%.
Fig. 4: Restriction map of the genomic region which includes the *phacA* gene of *A. nidulans*, showing the fragments that were subcloned to generate pFG4A and pFG4B. The dark area is the 3 introns.
Fig. 5: Restriction map of the genomic region which includes the *pahA* gene of *P. chrysogenum*, showing the 2.55 kb *XhoI* fragment that was subcloned to generate pALP520. The dark area is the 3 introns.
Fig. 6: Restriction map and relevant characteristics of the pPhacA::argB⁺ inactivation construct. The dark area is the sequencing region which includes the *phacA* gene; The white area is the flanking zones before and after the *phacA* gene; The striped area is the *argB*^{*+*} region.
Fig. 7: Restriction map and relevant characteristics of the pALP696 inactivation construct. The dark area is the sequencing region which includes the *pahA* gene (1745 bp); The white area is the flanking zones before and after the *pahA* gene (4143 bp); The striped area is the fleomycin resistance region, gene *ble*^{*R*} (2048 bp).

LIST OF SEQUENCES

GENERAL INFORMATION:

APPLICANT:
NAME: ANTIBIOTICOS, S.A.U.
STREET: Avda. de Burgos, 8-A
CITY: Madrid
STATE OR PROVINCE: Madrid
COUNTRY: Spain
POST CODE: 28036
TELEPHONE: 91-3841200
FAX: 91-3841220

TITLE: "PROCEDURE FOR INACTIVATION OF GENES THAT CODE
FOR PHENYLACETATE CATABOLISM ENZYMES, PLASMIDS
INVOLVED, AND STRAINS TRANSFORMED WITH THEM".

NUMBER OF SEQUENCES: 4

ADDRESS FOR CORRESPONDENCE:
ADDRESSEE: ANTIBIOTICS, S.A.U.
STREET: Avda. de Burgos, 8-A
CITY: Madrid
STATE OR PROVINCE Madrid
COUNTRY: Spain
POST CODE: 28036

MACHINE-READABLE FORM:
TYPE OF MEDIUM: 3.5" DISK
COMPUTER: PC
OPERATING SYSTEM: WINDOWS
SOFTWARE: WORD

INFORMATION ON THE LAWYER/AGENT:
NAME: ALBERTO DE ELZABURU
REGISTRATION NUMBER: 232/1
REFERENCE/REGISTRATION NUMBER: PCT-42

TELECOMMUNICATIONS INFORMATION:
TELEPHONE: 91 7009400
FAX: 91 3193810
TELEX OR ELECTRONIC MAIL: elzaburu@elzaburu.es

INFORMATION FOR SEQ ID NO:1

CHARACTERISTICS OF THE SEQUENCE:
LENGTH: 1986 base pairs
TYPE: nucleotides
STRANDEDNESS: 2
CONFIGURATION: linear
TYPE OF MOLECULE: genomic DNA
HYPOTHETICAL: NO
ANTISENSE: NO
ORIGINAL SOURCE: Aspergillus nidulans
IMMEDIATE SOURCE: plasmid pPhacA::argB+
POSITION IN THE GENOME: unknown
CHARACTERISTICS:
NAME/KEY: coding sequence
SITUATION: 82...1810
OTHER INFORMATION: phacA gene

DESCRIPTION OF THE SEQUENCE: SEQ ID NO: 1

C CAGCCAGATA TAAAGGCCGT 21
CCTTAGACGA CTTGATCTTA CTCTGGTTTC TAAAAGTTCA CTGATTATCG CAAGGTATCC 81
ATG TCT CTT CAA ACA ATC GGG ATC GCC GCT GTC GCG GTG GTC TAT TTT 129
Met Ser Leu Gln Thr Ile Gly Ile Ala Ala Val Ala Val Val Tyr Phe
                5                   10                  15
CTC ATC CGC TAC TTC AAC CGC ACA GAC ATC CCA AAG ATC AAG GGT CTC 177
Leu Ile Arg Tyr Phe Asn Arg Thr Asp Ile Pro Lys Ile Lys Gly Leu
            20                  25                  30
CCT GAA GTT CCA GGC GTA CCG ATC TTT GGC AAC CTC ATC CAG CTC GGT 225
Pro Glu Val Pro Gly Val Pro Ile Phe Gly Asn Leu Ile Gln Leu Gly
        35                  40                  45
GAC CAG CAC GCG ACC GTA GCG CAG AAA TGG GCG AAG AAA TTT GGA CCT 273
Asp Gln His Ala Thr Val Ala Gln Lys Trp Ala Lys Lys Phe Gly Pro
    50                  55                  60
GTT TTC CAG GTT CGC ATG GGG AAT AAA GTGAGTTCAG TGTCTGCATT TGTAAC 326
Val Phe Gln Val Arg Met Gly Asn Lys
65                  70

AGAC GACAATATTG CGAGAATAAT TCTGACCTAA CACAG CGC GTT GTC TTC GCA 380
Arg Val Val Phe Ala
    75

AAC ACC TTC GAC TCT GTC CGT CAG CTA TGG ATC AAA GAT CAG TCC GCG 428
Asn Thr Phe Asp Ser Val Arg Gln Leu Trp Ile Lys Asp Gln Ser Ala
    80                  85                  90

CTC ATC TCC CGG CCG ACC TTT CAC ACC TTC CAC AGT GTA GTT TCC AGC 476
Leu Ile Ser Arg Pro Thr Phe His Thr Phe His Ser Val Val Ser Ser
95                  100                 105                 110

TCT CAG GGA TTC ACC ATC GGA ACG TCG CCG TGG GAC GAG TCG TGC AAG 524
Ser Gln Gly Phe Thr Ile Gly Thr Ser Pro Trp Asp Glu Ser Cys Lys
                115                 120                 125

CGT CGT CGG AAG GCT GCA GCT ACA GCC TTG AAC CGC CCG GCT ACC CAG 572
Arg Arg Arg Lys Ala Ala Ala Thr Ala Leu Asn Arg Pro Ala Thr Gln
            130                 135                 140

TCG TAT ATG CCT ATT ATC GAT CTT GAG TCG ATG TCG AGT ATC CGG GAA 620
Ser Tyr Met Pro Ile Ile Asp Leu Glu Ser Met Ser Ser Ile Arg Glu
        145                 150                 155

TTG CTC AGG GAT AGC GCG AAT GGA ACA ATG GAT ATC AAC CCG ACA GCG 668
Leu Leu Arg Asp Ser Ala Asn Gly Thr Met Asp Ile Asn Pro Thr Ala
    160                 165                 170

TAC TTC CAG CGG TTC GCG TTG AAC ACG AGC TTA ACA TTG AAC TAT GGA 716
Tyr Phe Gln Arg Phe Ala Leu Asn Thr Ser Leu Thr Leu Asn Tyr Gly
175                 180                 185                 190

ATC CGA ATC GAG GGC AAT GTG AAC GAT GAG CTT TTG CGC GAA ATT GTC 764
Ile Arg Ile Glu Gly Asn Val Asn Asp Glu Leu Leu Arg Glu Ile Val
                195                 200                 205

GAT GTC GAG CGC GGG GTG TCG AAC TTC CGG AGT ACC AGC AAC CAG TGG 812
Asp Val Glu Arg Gly Val Ser Asn Phe Arg Ser Thr Ser Asn Gln Trp
            210                 215                 220

CAG GAC TAT ATC CCG CTC CTG AGA ATC TTC CCG AAG ATG AAC CGC GAG 860
Gln Asp Tyr Ile Pro Leu Leu Arg Ile Phe Pro Lys Met Asn Arg Glu
        225                 230                 235

GCT GAG GAG TTC CGG GTG CGG AGA GAC AAG TAT CTT ACC TAT CTT TTG 908
Ala Glu Glu Phe Arg Val Arg Arg Asp Lys Tyr Leu Thr Tyr Leu Leu
    240                 245                 250

GAT GTT CTC AAG GAT CGC ATT GCA AAG GGA ACC GAC AAG CCC TGT ATT 956
Asp Val Leu Lys Asp Arg Ile Ala Lys Gly Thr Asp Lys Pro Cys Ile
255                 260                 265                 270

ACT GGA AAC ATC CTC AAA GAC CCT GAG GCT AAG CTC AAT GAT G GTATG 1004
Thr Gly Asn Ile Leu Lys Asp Pro Glu Ala Lys Leu Asn Asp
                275                 280

CCGTC CGGTCCTGTC CGCGCTTGAA GGAAAATAAA GATTAACAGA GTCTAG CC GAG 1060
Ala Glu
285
ATT AAA TCG ATC TGC TTG ACC ATG GTC TCC GCC GGC CTC GAT ACT GTT 1108
Ile Lys Ser Ile Cys Leu Thr Met Val Ser Ala Gly Leu Asp Thr Val
            290                 295                 300
CCG GGC AAC TTG ATC ATG GGC ATC GCG TAC CTC GCC TCC GAA GAC GGC 1156
Pro Gly Asn Leu Ile Met Gly Ile Ala Tyr Leu Ala Ser Glu Asp Gly
        305                 310                 315
CAA AGG ATC CAG AAG CGC GCC CAC GAC GAG ATC ATG AAA GTC TAC CCG 1204
Gln Arg Ile Gln Lys Arg Ala His Asp Glu Ile Met Lys Val Tyr Pro
    320                 325                 330
GAC GGC GAT GCA TGG GAG AAA TGC CTG CTC GAA GAG AAA GTC CCC TAC 1252
Asp Gly Asp Ala Trp Glu Lys Cys Leu Leu Glu Glu Lys Val Pro Tyr
335                 340                 345                 350
GTC ACA GCT TTG GTC AAA GAA ACC CTC CGC TTC TGG ACT GTC ATT CCC 1300
Val Thr Ala Leu Val Lys Glu Thr Leu Arg Phe Trp Thr Val Ile Pro
                355                 360                 365
ATC TGT CTG CCT AGA GAA AAC ACC AAG GAT ATT GTC TGG AAC GGA GCC 1348
Ile Cys Leu Pro Arg Glu Asn Thr Lys Asp Ile Val Trp Asn Gly Ala
            370                 375                 380
GTT ATC CCT AAG GGA ACG ACC TTT TTC ATG AAC GCC TAC GCT GCA GAC 1396
Val Ile Pro Lys Gly Thr Thr Phe Phe Met Asn Ala Tyr Ala Ala Asp
        385                 390                 395
TAC GAC GAA ACA CAC TTC ACC AAT CCA CAC GCC TTT GAA CCA GAA CGC 1444
Tyr Asp Glu Thr His Phe Thr Asn Pro His Ala Phe Glu Pro Glu Arg
    400                 405                 410
TAC CTC ACC GCC TCA TCT GAC GGC TCC GGC ACT CCA CAC TAC GGC TAC 1492
Tyr Leu Thr Ala Ser Ser Asp Gly Ser Gly Thr Pro His Tyr Gly Tyr
415                 420                 425                 430
GGC GCG GGC TCG CGC ATG GTACCTCCCC CACAACCCAC ATGTTATATA GACAAT 1546
Gly Ala Gly Ser Arg Met
                435
ACTG ATTTATTATG AAG TGC GCT GGC TCG CAT CTT GCG AAC CGC GAG CTC 1596
Cys Ala Gly Ser His Leu Ala Asn Arg Glu Leu
            440                 445
TTC ACC GCT TAC GTC CGT CTG ATC ACA GCA TTT ACC ATG CAT CCA GCT 1644
Phe Thr Ala Tyr Val Arg Leu Ile Thr Ala Phe Thr Met His Pro Ala
        450                 455                 460
AAG AGG GCT GAG GAT CGG CCG ATC CTC GAT GCG ATT GAG TGT AAT GCG 1692
Lys Arg Ala Glu Asp Arg Pro Ile Leu Asp Ala Ile Glu Cys Asn Ala
    465                 470                 475

ATC CCG ACC GCT TTG ACG ACG GAG CCG AAG CCC TTC AAG GTT GGG TTT 1740
Ile Pro Thr Ala Leu Thr Thr Glu Pro Lys Pro Phe Lys Val Gly Phe
480                 485                 490                 495
AAA CCG AGA GAT CCT GTC TTG GTA AGG AAA TGG ATT GCG GAG AGT GAG 1788
Lys Pro Arg Asp Pro Val Leu Val Arg Lys Trp Ile Ala Glu Ser Glu
                500                 505                 510
GAG AGG ACG AAG CAC CTG AAT TAGTTTTTCT TTTCTTTCTT GCGTGCAAGT TAC 1842
Glu Arg Thr Lys His Leu Asn
            515
AGCGCAATTT ATACTTAGCT GGGACATGGT CGATTGGAGT ATACTGATTT CAGCAACAGC 1902
GCAAATAAAA ATAAGGCAAC CAATAGAAAT GCAACAATAT GCAATCTCCC AAGACCATTA 1962
AATGGTTGAT CAGATGATCC CAAG 1986

INFORMATION FOR SEQ ID NO.: 2

CHARACTERISTICS OF THE SEQUENCE:
LENGTH: 518 amino acids
TYPE: amino acids
STRANDEDNESS: 1
CONFIGURATION: linear
TYPE OF MOLECULE: peptide
ORIGINAL SOURCE: Aspergillus nidulans
CHARACTERISTICS:
OTHER INFORMATION: amino acid sequence of the enzyme
phenylacetate 2-hydroxylase
CHARACTERISTICS:
OTHER INFORMATION: Molecular weight 58495 Da.

DESCRIPTION OF THE SEQUENCE: SEQ ID NO: 2

Met Ser Leu Gln Thr Ile Gly Ile Ala Ala Val Ala Val Val Tyr
                5                   10                  15
Phe Leu Ile Arg Tyr Phe Asn Arg Thr Asp Ile Pro Lys Ile Lys
                20                  25                  30
Gly Leu Pro Glu Val Pro Gly Val Pro Ile Phe Gly Asn Leu Ile
                35                  40                  45

Gln Leu Gly Asp Gln His Ala Thr Val Ala Gln Lys Trp Ala Lys
                50                  55                  60
Lys Phe Gly Pro Val Phe Gln Val Arg Met Gly Asn Lys Arg Val
                65                  70                  75
Val Phe Ala Asn Thr Phe Asp Ser Val Arg Gln Leu Trp Ile Lys
                80                  85                  90
Asp Gln Ser Ala Leu Ile Ser Arg Pro Thr Phe His Thr Phe His
                95                  100                 105
Ser Val Val Ser Ser Ser Gln Gly Phe Thr Ile Gly Thr Ser Pro
                110                 115                 120
Trp Asp Glu Ser Cys Lys Arg Arg Arg Lys Ala Ala Ala Thr Ala
                125                 130                 135
Leu Asn Arg Pro Ala Thr Gln Ser Tyr Met Pro Ile Ile Asp Leu
                140                 145                 150
Glu Ser Met Ser Ser Ile Arg Glu Leu Leu Arg Asp Ser Ala Asn
                155                 160                 165
Gly Thr Met Asp Ile Asn Pro Thr Ala Tyr Phe Gln Arg Phe Ala
                170                 175                 180
Leu Asn Thr Ser Leu Thr Leu Asn Tyr Gly Ile Arg Ile Glu Gly
                185                 190                 195
Asn Val Asn Asp Glu Leu Leu Arg Glu Ile Val Asp Val Glu Arg
                200                 205                 210
Gly Val Ser Asn Phe Arg Ser Thr Ser Asn Gln Trp Gln Asp Tyr
                215                 220                 225
Ile Pro Leu Leu Arg Ile Phe Pro Lys Met Asn Arg Glu Ala Glu
                230                 235                 240
Glu Phe Arg Val Arg Arg Asp Lys Tyr Leu Thr Tyr Leu Leu Asp
                245                 250                 255
Val Leu Lys Asp Arg Ile Ala Lys Gly Thr Asp Lys Pro Cys Ile
                260                 265                 270
Thr Gly Asn Ile Leu Lys Asp Pro Glu Ala Lys Leu Asn Asp Ala
                275                 280                 285
Glu Ile Lys Ser Ile Cys Leu Thr Met Val Ser Ala Gly Leu Asp
                290                 295                 300
Thr Val Pro Gly Asn Leu Ile Met Gly Ile Ala Tyr Leu Ala Ser
                305                 310                 315
Glu Asp Gly Gln Arg Ile Gln Lys Arg Ala His Asp Glu Ile Met
                320                 325                 330
Lys Val Tyr Pro Asp Gly Asp Ala Trp Glu Lys Cys Leu Leu Glu
                335                 340                 345
Glu Lys Val Pro Tyr Val Thr Ala Leu Val Lys Glu Thr Leu Arg
                350                 355                 360

Phe Trp Thr Val Ile Pro Ile Cys Leu Pro Arg Glu Asn Thr Lys
                365                 370                 375
Asp Ile Val Trp Asn Gly Ala Val Ile Pro Lys Gly Thr Thr Phe
                380                 385                 390
Phe Met Asn Ala Tyr Ala Ala Asp Tyr Asp Glu Thr His Phe Thr
                395                 400                 405
Asn Pro His Ala Phe Glu Pro Glu Arg Tyr Leu Thr Ala Ser Ser
                410                 415                 420
Asp Gly Ser Gly Thr Pro His Tyr Gly Tyr Gly Ala Gly Ser Arg
                425                 430                 435
Met Cys Ala Gly Ser His Leu Ala Asn Arg Glu Leu Phe Thr Ala
                440                 445                 450
Tyr Val Arg Leu Ile Thr Ala Phe Thr Met His Pro Ala Lys Arg
                455                 460                 465
Ala Glu Asp Arg Pro Ile Leu Asp Ala Ile Glu Cys Asn Ala Ile
                470                 475                 480
Pro Thr Ala Leu Thr Thr Glu Pro Lys Pro Phe Lys Val Gly Phe
                485                 490                 495
Lys Pro Arg Asp Pro Val Leu Val Arg Lys Trp Ile Ala Glu Ser
                500                 505                 510
Glu Glu Arg Thr Lys His Leu Asn
                515

INFORMATION FOR SEQ ID NO.: 3

CHARACTERISTICS OF THE SEQUENCE:
LENGTH: 2558 base pairs
TYPE: nucleotides
STRANDEDNESS: 2
CONFIGURATION: linear
TYPE OF MOLECULE: genomic DNA
HYPOTHETICAL: NO
ANTISENSE: NO
ORIGINAL SOURCE: Penicillium chrysogenum
IMMEDIATE SOURCE: plasmid pALP520
POSITION IN THE GENOME: unknown
CHARACTERISTICS:
NAME/KEY: coding sequence

SITUTATION: 371..2094
CHARACTERISTICS:
OTHER INFORMATION: pahA gene

DESCRIPTTION OF THE SEQUENCE: SEQ ID NO: 3

CTCGAGAAGG TCTGTTGACA CGAGTCCACA AGATGTTGCG AAGTATATAA TATGAGAAAT 60
GGATATACAG CGGAAAATTG AAGTTTCATT CCGCGGGGGC GGGGAACAAG AGGAAGCGCG 120
GAATGAACAT TCCGTCTATG CGCGGTGGAG AGATCTTCGA ACTGGGGACT TGTGGACTTG 180
GACCTATGAG AGAGCTGCCT TTACTGATTC TGGGACTTGT GGCCAATGAA ACATGTCATG 240
GATGTTACAG CTTTCTTTAT GCGACTCTCT AAACTCGGAA ATATGCCGTA GCCGAAATGC 300
AGGAAAGGCT GGGCTATAAG AGATGCATGC TCCCACCGAA CGTCTGCAAT TCCTTGTTTC 360
ACTCTATATC ATG GCC ATC CAA ACA CTC GCA GTC GCC GTG ATC ACG GTG GTC 412
Met Ala Ile Gln Thr Leu Ala Val Ala Val Ile Thr Val Val
                5                   10
TAT TTC GTC ATT CGA TAC TTC AAC CGC ACT GAT ATC CCT AAG ATT AAA 460
Tyr Phe Val Ile Arg Tyr Phe Asn Arg Thr Asp Ile Pro Lys Ile Lys
15                  20                  25                  30
GGC CTC CCG GAG ATT CCT GGT ATA CCC ATA TTT GGC AAT CTA TTG CAG 508
Gly Leu Pro Glu Ile Pro Gly Ile Pro Ile Phe Gly Asn Leu Leu Gln
                35                  40                  45
CTA GGA GAT CAA CAT GCC ACA GTC ACG GGG AAA TGG GCA AAG AAA TTT 556
Leu Gly Asp Gln His Ala Thr Val Thr Gly Lys Trp Ala Lys Lys Phe
            50                  55                  60
GGC CCA GTT TTC CAA GTG CGC ATG GGA AAC AAG GTAAGTAATC AAATAATCTT 609
Gly Pro Val Phe Gln Val Arg Met Gly Asn Lys
        65                  70
TTAAATCGGA CATTCTGATA ATCTAATGCC CTTTTAG CGC ATC GTG TTC GCC AAC 664
Arg Ile Val Phe Ala Asn
    75
GGC TTT GAC TCC GTT CGT CAA TTG TGG ATT AAG GAC TCG TCG GCT CTG 712
Gly Phe Asp Ser Val Arg Gln Leu Trp Ile Lys Asp Ser Ser Ala Leu
80                  85                  90                  95
ATC TCC CGC CCA ACT TTC CAC ACT TTC CAC AGC GTC GTC TCC AGT TCA 760
Ile Ser Arg Pro Thr Phe His Thr Phe His Ser Val Val Ser Ser Ser
                100                 105                 110
CAG GGC TTC ACG ATC GGA ACT TCC CCG TGG GAT GAT TCC TGT AAG AAA 808
Gln Gly Phe Thr Ile Gly Thr Ser Pro Trp Asp Asp Ser Cys Lys Lys
            115                 120                 125

CGT CGC AAG GCA GCT GCC ACT GCG CTG AAT CGA CCG GCC GTG CAA TCG 856
Arg Arg Lys Ala Ala Ala Thr Ala Leu Asn Arg Pro Ala Val Gln Ser
        130                 135                 140
TAT ATG CCC ATC ATC GAT CTT GAA TCC AAT TCC AGC ATC AAA GAA CTG 904
Tyr Met Pro Ile Ile Asp Leu Glu Ser Asn Ser Ser Ile Lys Glu Leu
    145                 150                 155
TAC CGG GAC AGC CAA AAT GGC AAA CGT GAT GTG AAT CCC ACT GCA TAC 952
Tyr Arg Asp Ser Gln Asn Gly Lys Arg Asp Val Asn Pro Thr Ala Tyr
160                 165                 170                 175
TTC CAG CGA TAC GCT TTC AAC ACC AGT TTG ACT TTG AAC TAT GGA TTC 1000
Phe Gln Arg Tyr Ala Phe Asn Thr Ser Leu Thr Leu Asn Tyr Gly Phe
                180                 185                 190
CGC ATC GAG GGC AAT GTG GAT GAT ACG CTG CTG CAT GAG ATT GTG GAT 1048
Arg Ile Glu Gly Asn Val Asp Asp Thr Leu Leu His Glu Ile Val Asp
            195                 200                 205
GTG GAG CGT GGT GTG TCC AAC TTC CGC AGC ACT TCG AAC AAC TGG CAG 1096
Val Glu Arg Gly Val Ser Asn Phe Arg Ser Thr Ser Asn Asn Trp Gln
        210                 215                 220
GAC TAC ATT CCC CTA TTG CGC ATT TTC CCC AAG ATG AAC AAT GAG GCC 1144
Asp Tyr Ile Pro Leu Leu Arg Ile Phe Pro Lys Met Asn Asn Glu Ala
    225                 230                 235
GCT GAC TTC CGG GGT CGC CGT GAT AAA TAT CTG ACC TAC TTG CTC GAT 1192
Ala Asp Phe Arg Gly Arg Arg Asp Lys Tyr Leu Thr Tyr Leu Leu Asp
240                 245                 250                 255
ATG CTC AAG GAT CGA ATC GCC AAG GGA ACC GAT AAG CCT TGC ATC ACT 1240
Met Leu Lys Asp Arg Ile Ala Lys Gly Thr Asp Lys Pro Cys Ile Thr
                260                 265                 270
GGT AAT ATC TTG AAG GAT CCC GAG GCT AAG CTG AAT GAT G GTGAGTTACA 1290
Gly Asn Ile Leu Lys Asp Pro Glu Ala Lys Leu Asn Asp
            275                 280
AAATCTCGCG AAATCTTTGT GAATGTTGGT ATTGAGTACT CATCTATCGT CTAG CC 1346
Ala
285
GAG GTG AAA TCG ATC TGT TTG ACT ATG GTG TCG GCT GGC CTT GAC ACC 1394
Glu Val Lys Ser Ile Cys Leu Thr Met Val Ser Ala Gly Leu Asp Thr
                290                 295                 300
GTT CCG GGC AAC TTG ATT ATG GGA ATT GCC TAC CTG GCA TCT GAA GAC 1442
Val Pro Gly Asn Leu Ile Met Gly Ile Ala Tyr Leu Ala Ser Glu Asp
            305                 310                 315
GGC CAA AGA ATT CAG AAA AAG GCC TAT GAT GCA ATC ATG GAG GTA TAC 1490
Gly Gln Arg Ile Gln Lys Lys Ala Tyr Asp Ala Ile Met Glu Val Tyr
        320                 325                 330

CCG GAC GGC GAT GCT TGG GAG AAA TGT TTG GTG GAG GAG AAG GTC CCT 1538
Pro Asp Gly Asp Ala Trp Glu Lys Cys Leu Val Glu Glu Lys Val Pro
    335                 340                 345
TAT GTG ACG GCA CTG GTC AAG GAG GTC TTG CGC TTC TGG ACG GTC ATT 1586
Tyr Val Thr Ala Leu Val Lys Glu Val Leu Arg Phe Trp Thr Val Ile
350                 355                 360                 365
CCT ATT TGT CTG CCA CGC GAG AGC ACC AAG GAT ATC CAG TGG AAT GGA 1634
Pro Ile Cys Leu Pro Arg Glu Ser Thr Lys Asp Ile Gln Trp Asn Gly
                370                 375                 380
GCT ACA ATC CCT GCC GGG ACG ACC TTT TTC ATG GTATGTTGCG CCTTGCTTCT 1687
Ala Thr Ile Pro Ala Gly Thr Thr Phe Phe Met
            385                 390
GTCCCTTTCG GGACGTTGCT AACAGGATCT GATAG AAT GTT TGG GCT GCC GAT 1740
Asn Val Trp Ala Ala Asp
        395
TAC GAT GAA GAT CAC TTT AAG GAT GCC GAT AAA TTC ATC CCG GAG CGT 1788
Tyr Asp Glu Asp His Phe Lys Asp Ala Asp Lys Phe Ile Pro Glu Arg
    400                 405                 410
TAT TTG GAG GCC AGT GAG GGT GCA GGC ACT CCA CAC TAT GCA TAT GGA 1836
Tyr Leu Glu Ala Ser Glu Gly Ala Gly Thr Pro His Tyr Ala Tyr Gly
415                 420                 425                 430
GCG GGA TCA CGT ATG TGT GCA GGC TCA CAT CTC GCC AAT CGC GAG CTG 1884
Ala Gly Ser Arg Met Cys Ala Gly Ser His Leu Ala Asn Arg Glu Leu
                435                 440                 445
TTC ACT GCT TTT ATC CGC CTC GTC ACT GCT TTC AAC ATG CAC ACG GCG 1932
Phe Thr Ala Phe Ile Arg Leu Val Thr Ala Phe Asn Met His Thr Ala
            450                 455                 460
AAG GAG ACG GCC GAC CGA CCG ATT CTG AAT GCA ATT GAG TGC AAT TTG 1980
Lys Glu Thr Ala Asp Arg Pro Ile Leu Asn Ala Ile Glu Cys Asn Leu
        465                 470                 475
ATT CCG ACA GCC TTG ACA ACC GAG CCG AAG CCA TTC AAG GTT GGC TTT 2028
Ile Pro Thr Ala Leu Thr Thr Glu Pro Lys Pro Phe Lys Val Gly Phe
    480                 485                 490
AGT GCA CGC GAC CCT AAG AAG CTT GAG CAG TGG ATT GCT GAG AGC GAT 2076
Ser Ala Arg Asp Pro Lys Lys Leu Glu Gln Trp Ile Ala Glu Ser Asp
495                 500                 505                 510
GAA CGG ACC AAA GAT CTA TAAGAGGAAG TTTTATCTGA TATGATTCCC TTTTTTTTTT
Glu Arg Thr Lys Asp Leu
                515
TCGGAGGCTA TTTATGTATC TACTTGAATA TATGTGAAAT AAAGGCAATG AAGTATAGAT 2194
ATAGACCTGC CCAGGTGAGA CCTACATGTA TGTAATCGAC GTCTCCCAGT ACCTATTTAG 2254
GAACCGAATA GAAATTTGAG TGATGAAGGG TGAAGAATAA CCTCAGAGAA CATAGGTCTA 2314

CTAAGATCTG CTAATTCTTA GACTCCTTTC CCTGAATTAT TGCCAATTTC CCCAGTTGTC 2374
TCTCTCCTCT ATGACTCCCG GCCTTGTCAC ACCGGCGGAA TCTCCCCGCA TTTTCCTCGA 2434
CCTCACCCTT TTCTTCCTCT TCACCCTCTC CCCATCCACT TGAAATGAAC CTCTGATCGC 2494
AATCAATTGC ATCCCGAAGT CATTTTGGAT GATGAATAAT CCGCAGATGT ACCCTGTCCT 2554
CGAG 2558

INFORMATION FOR SEQ ID NO.: 4

CHARACTERISTICS OF THE SEQUENCE:
LENGTH: 516 base pairs
TYPE: amino acids
STRANDEDNESS: 1
CONFIGURATION: linear
TYPE OF MOLECULE: peptide
CHARACTERISTICS:
OTHER INFORMATION: amino acid sequence of the enzyme
phenylacetate 2-hydroxylase
CHARACTERISTICS:
OTHER INFORMATION: Molecular weight 58112 Da.

DESCRIPTION OF THE SEQUENCE: SEQ ID NO: 4

Met Ala Ile Gln Thr Leu Ala Val Ala Val Ile Thr Val Val Tyr Phe
1               5                   10                  15
Val Ile Arg Tyr Phe Asn Arg Thr Asp Ile Pro Lys Ile Lys Gly Leu
            20                  25                  30
Pro Glu Ile Pro Gly Ile Pro Ile Phe Gly Asn Leu Leu Gln Leu Gly
        35                  40                  45
Asp Gln His Ala Thr Val Thr Gly Lys Trp Ala Lys Lys Phe Gly Pro
    50                  55                  60
Val Phe Gln Val Arg Met Gly Asn Lys Arg Ile Val Phe Ala Asn Gly
65                  70                  75                  80
Phe Asp Ser Val Arg Gln Leu Trp Ile Lys Asp Ser Ser Ala Leu Ile
                85                  90                  95
Ser Arg Pro Thr Phe His Thr Phe His Ser Val Val Ser Ser Ser Gln
            100                 105                 110
Gly Phe Thr Ile Gly Thr Ser Pro Trp Asp Asp Ser Cys Lys Lys Arg
        115                 120                 125

Arg Lys Ala Ala Ala Thr Ala Leu Asn Arg Pro Ala Val Gln Ser Tyr
    130                 135                 140
Met Pro Ile Ile Asp Leu Glu Ser Asn Ser Ser Ile Lys Glu Leu Tyr
145                 150                 155                 160
Arg Asp Ser Gln Asn Gly Lys Arg Asp Val Asn Pro Thr Ala Tyr Phe
                165                 170                 175
Gln Arg Tyr Ala Phe Asn Thr Ser Leu Thr Leu Asn Tyr Gly Phe Arg
            180                 185                 190
Ile Glu Gly Asn Val Asp Asp Thr Leu Leu His Glu Ile Val Asp Val
        195                 200                 205
Glu Arg Gly Val Ser Asn Phe Arg Ser Thr Ser Asn Asn Trp Gln Asp
    210                 215                 220
Tyr Ile Pro Leu Leu Arg Ile Phe Pro Lys Met Asn Asn Glu Ala Ala
225                 230                 235                 240
Asp Phe Arg Gly Arg Arg Asp Lys Tyr Leu Thr Tyr Leu Leu Asp Met
                245                 250                 255
Leu Lys Asp Arg Ile Ala Lys Gly Thr Asp Lys Pro Cys Ile Thr Gly
            260                 265                 270
Asn Ile Leu Lys Asp Pro Glu Ala Lys Leu Asn Asp Ala Glu Val Lys
        275                 280                 285
Ser Ile Cys Leu Thr Met Val Ser Ala Gly Leu Asp Thr Val Pro Gly
    290                 295                 300
Asn Leu Ile Met Gly Ile Ala Tyr Leu Ala Ser Glu Asp Gly Gln Arg
305                 310                 315                 320
Ile Gln Lys Lys Ala Tyr Asp Ala Ile Met Glu Val Tyr Pro Asp Gly
                325                 330                 335
Asp Ala Trp Glu Lys Cys Leu Val Glu Glu Lys Val Pro Tyr Val Thr
            340                 345                 350
Ala Leu Val Lys Glu Val Leu Arg Phe Trp Thr Val Ile Pro Ile Cys
        355                 360                 365
Leu Pro Arg Glu Ser Thr Lys Asp Ile Gln Trp Asn Gly Ala Thr Ile
    370                 375                 380
Pro Ala Gly Thr Thr Phe Phe Met Asn Val Trp Ala Ala Asp Tyr Asp
385                 390                 395                 400
Glu Asp His Phe Lys Asp Ala Asp Lys Phe Ile Pro Glu Arg Tyr Leu
                405                 410                 415
Glu Ala Ser Glu Gly Ala Gly Thr Pro His Tyr Ala Tyr Gly Ala Gly
            420                 425                 430
Ser Arg Met Cys Ala Gly Ser His Leu Ala Asn Arg Glu Leu Phe Thr
        435                 440                 445
Ala Phe Ile Arg Leu Val Thr Ala Phe Asn Met His Thr Ala Lys Glu
    450                 455                 460

Thr Ala Asp Arg Pro Ile Leu Asn Ala Ile Glu Cys Asn Leu Ile Pro
465                 470                 475                 480
Thr Ala Leu Thr Thr Glu Pro Lys Pro Phe Lys Val Gly Phe Ser Ala
                485                 490                 495
Arg Asp Pro Lys Lys Leu Glu Gln Trp Ile Ala Glu Ser Asp Glu Arg
            500                 505                 510
Thr Lys Asp Leu
        515

## Claims

1. Procedure for the inactivation, in microorganisms, of genes which code for phenylacetate catabolism enzymes and which compete for this compound with the penicillin biosynthesis enzymes, consisting in an integrative transformation by homologous recombination between at least one exogenous DNA compound and at least part of the sequence of the gene which is to be inactivated.

2. Procedure according to Claim 1, in which the transformant DNA compound is a circular molecule which contains at least one expressible fragment of the gene which is to be inactivated.

3. Procedure according to Claim 1, in which the transformant DNA compound is a linear molecule which contains at least one DNA fragment not included in the sequence of the gene to be inactivated, but which contains at least one transformation marker that interrupts the coding sequence thereof.

4. Procedure according to the preceding claims, in which the transformant DNA molecule contains, wholly or in part, a copy of the sequence of the gene which is to be inactivated with a mutation, preferably a reading frame shift mutation, without sense or deletion, which results in a loss of function phenotype of the said gene.

5. Procedure according to any of the preceding claims, in which the microorganism that is the object of the transformation in which the gene is inactivated is able to produce penicillin G or V.

6. Procedure according to Claim 5, in which the microorganism producing penicillin G or V is a fungus.

7. Procedure according to Claims 1 to 6, in which the microorganism transformed is *Aspergillus nidulans.*

8. Procedure according to Claims 1 to 6, in which the microorganism transformed is *Penicillium chrysogenum*

9. Procedure according to Claims 1 to 7, in which the transformant DNA compound used can be included, wholly or in part, in a vector, preferably in the plasmid pPhacA::argB.

10. Procedure according to Claims 1 to 6 and 8, in which the transformant DNA compound used can be included, wholly or in part, in a vector, preferably in the plasmid pALP696.

11. Procedure according to Claims 1 to 7 and 9, in which the gene which is inactivated is represented by SEQ ID NO: 1, its mutated gene sequences and/or homologous genes.

12. Procedure according to Claim 11, in which the gene which is inactivated codes for a polypeptide represented by SEQ ID NO: 2, or similar sequences which express P450 phenylacetate 2-hydroxylase activity.

13. Procedure according to Claims 1 to 6, 8 and 10, in which the gene which is inactivated is represented by SEQ ID NO: 3, its mutated gene sequences and/or homologous genes.

14. Procedure according to Claim 13, in which the gene which is inactivated codes for a polypeptide represented by SEQ ID NO: 4, or similar sequences which express P450 phenylacetate 2-hydroxylase activity.

15. Transformed strain of *Aspergillus nidulans* and mutants derived therefrom which incorporates at least one exogenous DNA compound that consists in a truncated, incomplete or inactive sequence of at least one gene which codes for a phenylacetate catabolism enzyme and which inactivates the endogenous gene after its integration by homologous recombination.

16. Transformed strain of *A. nidulans* according to Claim 15, in which the gene to be inactivated codes for an enzyme that mediates the hydroxylation of phenylacetate.

17. Transformed strain of *A. nidulans* according to Claims 15 and 16, in which the gene that is inactivated is *phacA*, the sequence and flanking regions of which are described in SEQ ID NO: 1, its mutated gene sequences and homologous genes.

18. Transformed strain of *A. nidulans* according to Claim 17, in which the gene that is inactivated is homologous to *phacA*, of different origin than *A. nidulans* and the homology of which with the DNA sequence of the *phacA* gene of *A. nidulans* is sufficient to mediate homologous recombination with the endogenous locus of *A. nidulans*.

19. Transformed strain of *A. nidulans* according to Claims 15 to 18, in which the gene which is inactivated codes for a polypeptide represented by the protein sequence SEQ ID NO: 2, or similar sequences which express P450 phenylacetate 2-hydroxylase activity.

20. Transformed strain according to Claims 15 to 19, characterized in that it consists in a pure strain of *A. nidulans* CECT20195, its mutants and/or transformed derivatives.

21. Plasmid pPhacA::argB inactivating the *phcA* gene of *A. nidulans*, as shown in the restriction map in Fig. 6, and which consists in the plasmid pUC18 that contains a 6.8 kb *Eco*RI insert which includes the *phacA* gene of *A. nidulans* inactivated by means of the insertion of a 3.2 kb fragment which in turn contains the *argB* gene of *A. nidulans*.

22. Transformed strain of *Penicillium chrysogenum* and mutants derived therefrom which contains a DNA compound that consists in a truncated, incomplete or inactive sequence of a gene which codes for a phenylacetate catabolism enzyme and which inactivates the endogenous gene after its integration by homologous recombination.

23. Transformed strain of *P. chrysogenum* according to Claim 22, in which the gene to be inactivated codes for an enzyme that mediates the hydroxylation of phenylacetate.

24. Transformed strain of *P. chrysogenum* according to Claims 22 and 23, in which the gene is *phcA*, the sequence of which and that of its flanking regions are described in SEQ ID NO: 3, its mutated gene sequences and/or homologous genes.

25. Transformed strain of *P. chrysogenum* according to Claims 22 to 24, in which the gene which is inactivated codes for a polypeptide represented by the protein sequence SEQ ID NO: 4, or similar sequences which express definite P450 phenylacetate 2-hydroxylase activity.

26. Transformed strain of *P. chrysogenum* according to Claim 24, in which the gene is *phacA* of *A. nidulans* or any other homologous DNA compound isolated from a source different from *P. chrysogenum* and the homology of which with the DNA sequence of *pahA* of *P. chrysogenum* is sufficient to mediate homologous recombination with the endogenous locus of *P. chrysogenum.*

27. Plasmid pALP696 inactivating the *pahA* gene of *P. chrysogenum*, as shown in the restriction map in Fig. 7, and which consists in the plasmid pBC KS+ that contains a 7.9 kb *Sal*I insert which includes the *pahA* gene of *P. chrysogenum* inactivated by means of the insertion of a 2.0 kb fragment which in turn contains the *ble*^{*R*} gene of *S. hindustanus* expressed under the control of the *gdh* promoter of *P. chrysogenum.*
